(19) Europäisches Patentamt
European Patent Office
Office européen des brevets

(11) EP 2 606 935 A2

(12) EUROPÄISCHE PATENTANMELDUNG

(43) Veröffentlichungstag:
**26.06.2013 Patentblatt 2013/26**

(21) Anmeldenummer: **12195795.5**

(22) Anmeldetag: **06.12.2012**

(51) Int Cl.:
***A61Q 5/10*** *(2006.01)*
***A61K 8/49*** *(2006.01)*
***A61K 8/44*** *(2006.01)*
***A61Q 5/12*** *(2006.01)*
***A61K 8/92*** *(2006.01)*
***A61K 36/61*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **19.12.2011 DE 102011089042**

(71) Anmelder: **Henkel AG&Co. KGAA**
**40589 Düsseldorf (DE)**

(72) Erfinder:
- **Reichert, Anja**
  **40629 Düsseldorf (DE)**
- **Babiel, Sabine**
  **47447 Moers (DE)**
- **Rohland, Christa**
  **40468 Düsseldorf (DE)**
- **Ehlert, Manuela**
  **51379 Leverkusen (DE)**

# (54) Luftoxidative Haarfärbemittel für glänzende Färbungen

(57) Gegenstand der Erfindung ist ein Mittel zur durch Luftsauerstoff induzierten Färbung keratinhaltiger Fasern, insbesondere menschlicher Haare, welches in einem kosmetisch akzeptablen Träger bei einem alkalischen pH-Wert von 8 bis 10,5 mindestens eine Verbindung der Formel (I),

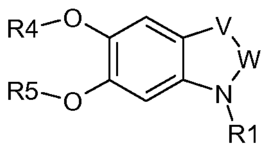
(I),

und mindestens ein pflanzliches Öl auf Basis eines Fettsäuretriglyceridgemischs (B) enthält, wobei neben Luftsauerstoff kein zusätzliches Oxidationsmittel enthalten ist, und wobei das Fettsäuretriglyceridgemisch (B) einen Anteil von mindestens 25 Gew.-% Palmitoleinsäure, bezogen auf das Gesamtgewicht aller Fettsäuren des Fettsäuretriglyceridgemisches (B), aufweist.

EP 2 606 935 A2

## Beschreibung

Gegenstand der Erfindung ist ein Mittel zur durch Luftsauerstoff induzierten Farbveränderung von keratinischen Fasern, welches in einem kosmetischen Träger neben mindestens einer speziellen, durch Luftsauerstoff farbgebenden Verbindung ein pflanzliches Öl auf Basis eines Triglyceridgemischs mit einem bestimmten Anteil an Palmitoleinsäure enthält.

Die Veränderung von Farbe der Haare stellt einen wichtigen Bereich der modernen Kosmetik dar. Neben Oxidationsfärbemitteln für permanente, intensive Färbungen mit entsprechenden Echtheitseigenschaften, bei denen die Färbung durch Zusatz von Oxidationsmittel, wie Wasserstoffperoxid initiiert wird, und Färbe- oder Tönungsmittel, die als färbende Komponente sogenannte direktziehende Farbstoffe enthalten und keinen oxidativen Prozess zur Ausbildung der Farbe benötigen, hat ein weiteres Färbeverfahren große Beachtung gefunden, bei dem Vorstufen des natürlichen Haarfarbstoffes Melanin auf das Haar aufgebracht werden, wobei diese dann im Rahmen oxidativer Prozesse im Haar naturanaloge Farbstoffe ausbilden.

Diese Färbungen können sich bereits unter Oxidation durch Luftsauerstoff als einzigem Oxidationsmittel erfolgen, so dass auf keine weiteren zusätzlichen Oxidationsmittel zurückgegriffen werden muss. Daher sind solche Färbungen besonders schonend für die Haarstruktur. Die Mittel erlauben es bei, insbesondere mehrfacher, Anwendung, Menschen mit ergrauten Haaren die natürliche Haarfarbe wiederzugeben. Die Färbeleistungen der Mittel sind jedoch vielfach, und insbesondere bei Luftsauerstoffoxidation, nicht zufriedenstellend und erfordern lange Einwirkzeiten oder häufige Wiederholungen des Färbevorgangs. Weiterhin laufen auch diese Färbungen in der Regel im alkalischen Medium ab, so dass die Haarfaser durch den Einsatz, insbesondere aufgrund der langen Einwirkzeiten und der Wiederholungen, strapaziert wird und entsprechende optische und haptische Defizite aufweist.

Es ist daher besonders wünschenswert, luftoxidative Färbemittel bereitzustellen, die neben einer guten Färbeleistung einen positiven Beitrag zur Verbesserung der Faserstruktur leisten und damit gleichzeitig als Pflege- oder Konditioniermittel wirken. Insbesondere eine Verbesserung des Glanzes der Faser nach der Färbung ist dabei erstrebenswert, um den optischen Eindruck der Färbung zu verbessern.

Aufgabe der vorliegenden Erfindung ist daher die Bereitstellung kosmetischer Mittel auf Basis von Luftoxidation Färbemitteln zur Farbveränderung keratinischer Fasern, die die oben beschriebenen Nachteile ausgleichen und die einen verbesserten Glanz der gefärbten Fasern aufweisen.

In nicht vorhersehbarer Weise konnte nun gefunden werden, dass farbverändernde Mittel, die bei einem alkalischen pH-Wert neben einer bestimmten farbverändernden Komponente mindestens ein Fettsäuretriglyceridgemisch mit einem bestimmten Anteil an Palmitoleinsäure enthalten, die oben genannten Nachteile vermeiden. Bedingt durch die pflegende Wirkung bei Anwendung des erfindungsgemäßen Mittels kann der Glanz der behandelten Haare hierdurch deutlich verbessert werden.

Ein erster Gegenstand der Erfindung ist daher ein Mittel zur durch Luftsauerstoff induzierten Färbung keratinhaltiger Fasern, insbesondere menschlicher Haare, welches in einem kosmetisch akzeptablen Träger bei einem alkalischen pH-Wert von 8 bis 10,5

(a) mindestens eine Verbindung der Formel (I),

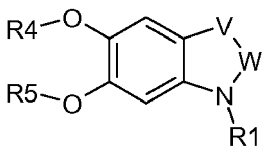

(I),

worin unabhängig voneinander

- R1 für Wasserstoff, eine $C_1$-$C_4$-Alkylgruppe oder eine $C_2$-$C_4$-Hydroxyalkylgruppe steht,
- V für CHR3 und W für CHR2 stehen,
  so dass die Bindung zwischen V und W einer Einfachbindung entspricht,
- oder V für CR3 und W für CR2 stehen,
  so dass die Bindung zwischen V und W einer Doppelbindung entspricht,
  wobei jeweils
- R2 für Wasserstoff oder eine -COOH-Gruppe steht,
  wobei die -COOH-Gruppe auch als Salz mit einem physiologisch verträglichen Kation vorliegen kann,
- R3 für Wasserstoff oder eine $C_1$-$C_4$-Alkylgruppe steht,
- R4 und R5 unabhängig voneinander für Wasserstoff, eine $C_1$-$C_4$-Alkylgruppe oder eine Gruppe -CO-R6 stehen,

in der R6 für eine $C_1$-$C_4$-Alkylgruppe steht,

und/oder eines deren physiologisch verträgliche Salze mit einer organischen oder anorganischen Säure,
(b) und mindestens ein pflanzliches Öl auf Basis eines Fettsäuretriglyceridgemischs (B) enthält, und welches dadurch gekennzeichnet ist, dass
neben Luftsauerstoff kein zusätzliches Oxidationsmittel enthalten ist, und dass das Fettsäuretriglyceridgemisch (B) einen Anteil von mindestens 25 Gew.-% Palmitoleinsäure, bezogen auf Gesamtgewicht aller Fettsäuren des Fettsäuretriglyceridgemisches (B), aufweist.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die Mittel enthalten neben Luftsauerstoff, mit dem sie bei Herstellung, Lagerung oder Anwendung in Kontakt kommen, kein weiteres zusätzliches Oxidationsmittel, insbesondere jedoch keine Peroxoverbindungen wie Wasserstoffperoxid, Persulfate, Peroxomonosulfate, Perborate, Chlorite, Hypochlorite oder Peroxidsalze.

Die erfindungsgemäßen Mittel enthalten als zusätzliche Oxidationsmittel insbesondere auch keine oxidativ wirksamen Enzyme, wie Peroxidasen, Laccasen oder sogenannte 2-Elektronen-Oxidoreduktasen in Kombination mit den dafür spezifischen Substraten, wie z. B. Pyranose-Oxidase und z.B. D-Glucose oder Galactose, Glucose-Oxidase und D-Glucose, Glycerin-Oxidase und Glycerin, Pyruvat-Oxidase und Benztraubensäure oder deren Salze, Alkohol-Oxidase und Alkohol (MeOH, EtOH), Lactat-Oxidase und Milchsäure und deren Salze, Tyrosinase-Oxidase und Tyrosin, Uricase und Harnsäure oder deren Salze sowie Cholinoxidase und Cholin sowie Aminosäure-Oxidase und entsprechende Aminosäuren.

Die erfindungsgemäßen Mittel enthalten die Wirkstoffe in einem kosmetischen Träger. Dieser kosmetische Träger ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, treibgasfreie Schäume, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthalten die erfindungsgemäßen Mittel mindestens eine Verbindung der Formel (I) als Farbstoffvorprodukt eines naturanalogen Farbstoffs.

Beispiele für $C_1$-$C_4$-Alkylgruppen in Verbindungen der Formel (I) sind Methyl, Ethyl, n-Propyl, i-Propyl, n-Butyl, s-Butyl, i-Butyl und t-Butyl.

Beispiele für $C_2$-$C_4$-Hydroxyalkylgruppen in Verbindungen der Formel (I) sind 2-Hydroxyethyl, 2-Hydroxypropyl, 3-Hydroxypropyl, 2-Hydroxybutyl, 3-Hydroxybutyl und 4-Hydroxybutyl.

Wenn in der Formel (I) V für eine Gruppe CR3 und W für eine Gruppe CR2, so dass die Bindung zwischen V und W einer Doppelbindung entspricht, so handelt es sich bei der Verbindung der Formel (I) um ein sogenanntes Indol-Derivat.

Solche Verbindungen stellen erfindungsgemäß besonders geeignete, luftoxidative Farbstoffvorstufen dar. Erfindungsgemäß besonders geeignete Verbidnungen der Formel (I) sind daher Indolderivate, insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure und/oder eines deren physiologisch verträgliche Salze mit einer organischen oder anorganischen Säure. Wenn in der Formel (I) V für eine Gruppe CHR3 und W für eine Gruppe CHR2, so dass die Bindung zwischen V und W einer Einfachbindung entspricht, so handelt es sich bei der Verbindung der Formel (I) um ein sogenanntes Indolin-Derivat.

Besonders gut als Vorstufen naturanaloger Haarfarbstoffe geeignet sind Derivate des 5,6-Dihydroxyindolins. Eine Ausführungsform des ersten Erfindungsgegenstands ist daher dadurch gekennzeichnet, dass die Verbindungen der Formel (I) ausgewählt werden aus Verbindungen der Formel (Ia) (Derivaten des 5,6-Dihydroxyindolins),

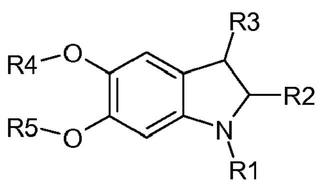

(Ia),

worin unabhängig voneinander

- R1 für Wasserstoff, eine $C_1$-$C_4$-Alkylgruppe oder eine $C_1$-$C_4$-Hydroxyalkylgruppe steht,
- R2 für Wasserstoff oder eine -COOH-Gruppe steht, wobei die -COOH-Gruppe auch als Salz mit einem physiologisch verträglichen Kation vorliegen kann,
- R3 für Wasserstoff oder eine $C_1$-$C_4$-Alkylgruppe steht,
- R4 und R5 unabhängig voneinander für Wasserstoff, eine $C_1$-$C_4$-Alkylgruppe oder eine Gruppe -CO-R6, in der R6 steht für eine $C_1$-$C_4$-Alkylgruppe,

und/oder eines deren physiologisch verträgliche Salze mit einer organischen oder anorganischen Säure.

Eine weitere Ausführungsform des ersten Erfindungsgegenstands ist dabei dadurch gekennzeichnet, dass die Verbindungen der Formel (I) ausgewählt werden aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure und/oder deren physiologisch verträglichen Salzen mit einer organischen oder anorganischen Säure.

Besonders bevorzugt ist insbesondere 5,6-Dihydroxyindolin und/oder deren physiologisch verträglichen Salzen mit einer organischen oder anorganischen Säure.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Die Verbindungen der Formel (I) sind als Vorstufen naturanaloger Farbstoffe in den erfindungsgemäßen Mitteln bevorzugt in einem Anteil von 0,01 bis 10 Gew.-%, insbesondere von 0,1 bis 5 Gew.-%, bevorzugt 0,1 bis 2,0 Gew.-% und besonders bevorzugt 0,6 bis 1,2 Gew.-%, jeweils bezogen auf das Gesamtgewicht des Mittels, enthalten.

Als weiteren wesentlichen Inhaltsstoff enthält das erfindungsgemäße Mittel mindestens ein pflanzliches Öl auf Basis eines Fettsäuretriglyceridgemischs (B), welches einen Anteil von mindestens 25 Gew.-% Palmitoleinsäure, bezogen auf Gesamtgewicht aller Fettsäuren des Fettsäuretriglyceridgemisches (B), aufweist.

Pflanzliche Öle bezeichnen hydrophobe, bei Raumtemperatur und Normaldruck flüssige oder wachsartige Produkte, welche aus Pflanzen gewonnen werden. Ein pflanzliches Öl auf Basis eines Fettsäuretriglyceridgemischs ist erfindungsgemäß als ein pflanzliches Öl zu verstehen, welches zum überwiegenden Gewichtsanteil aus Fettsäuretriglyceriden, insbesondere zu mehr als 90 Gew.-%, weiter zu mehr als 95 Gew.-% und besonders zu mehr als 98 Gew.-% aus Fettsäuretriglyceriden besteht. Der verbleibende Rest des Öls wird auch als unverseifbares bezeichnet.

Unter einem Fettsäuretriglycerid ist erfindungsgemäß ein Ester des Triols Glycerin mit drei Äquivalenten von Fettsäure zu verstehen. Dabei können sowohl strukturgleiche wie unterschiedliche Fettsäuren in einem Molekül Triglycerid an der Esterbildung beteiligt sein.

Insbesondere natürliche, und daher auch pflanzliche, Fettsäuretriglyceride stellen üblicherweise Gemische von Estern des Glycerins mit unterschiedlichen Fettsäuren dar. Üblicherweise sind solche Fettsäuretriglyceride gemischte Glyceride von gesättigten und ungesättigten Fettsäuren. Das erfindungsgemäße Mittel enthält daher ein Fettsäuretriglyceridgemisch (B).

Die erfindungsgemäßen Fettsäuretriglyceridgemische zeichnen sich dadurch aus, dass sie einen bestimmten Anteil an der ungesättigten Fettsäure Palmitoleinsäure enthalten. Dieser Anteil wird üblicherweise nach Verseifung des Esters und Analyse der freigesetzten Fettsäuren bestimmt. Dabei können unterschiedliche Methoden eingesetzt werden, wie beispielsweise Gaschromatographie- oder Hochdruckflüssigkeitschromatographie-Verfahren, wobei es zweckmäßig sein kann, die freie Fettsäure zu derivatisieren. Geeignete Derivate sind insbesondere Ester, wie Methylester. Unter einer ungesättigten Fettsäure sind erfindungsgemäß ungesättigte, gegebenenfalls verzweigte und/oder substituierte $C_8$-$C_{24}$-Fettsäuren zu verstehen. Bevorzugt handelt es sich bei der ungesättigten Fettsäure um eine Fettsäure, deren C-C-Doppelbindung und/oder C-C-Doppelbindungen die Cis-Konfiguration aufweisen. Beispiele für ungesättigte Fettsäuren in den erfindungsgemäß geeignete Triglyceridgemischen (A) sind Decensäure, Dodecensäure, Tetradecensäure, Palmitoleinsäure, Ölsäure, Petroselinsäure, Elaidinsäure, Linolsäure, Linolensäure, Arachidonsäure, Gadoleinsäure (Eicosensäure), Erucasäure (Docosensäure) und Nervonsäure (Tetracosensäure) sowie deren Mischungen.

Erfindungswesentlich ist dabei, dass das Fettsäuretriglyceridgemisch (B) Palmitoleinsäure ((9Z)-Hexadec-9-ensäure; C16:1; d.h. Fettsäure mit 16 Kohlenstoffatomen und einer C-C-Doppelbindung) in einem bestimmten Mindestanteil, bezogen auf das Gesamtgewicht der Fettsäuren, enthält. Bevorzugt ist Palmitoleinsäure im erfindungsgemäßen Fettsäuretriglyceridgemisch (A) in einem Anteil von 20 bis 45 Gew.-%, bevorzugt von 25 bis 40 Gew.-% und weiter bevorzugt von 30 bis 35 Gew.-%, jeweils bezogen auf das Gesamtgewicht aller Fettsäuren im Triglyceridgemisch (A), enthalten.

Zur Bestimmung des Anteils ungesättigter Fettsäuren in den Fettsäuretriglyceridgemischen sind dem Fachmann unterschiedliche Methoden bekannt. Ein besonders geeigneter Parameter für die Ermittelung erfindungsgemäß einsetzbarer Fettsäuretriglyceridgemische (B) stellt die Iodzahl dar.

Die Iodzahl ist dabei an Maßzahl für den Grad der Ungesättigtheit einer Verbindung und gibt die Menge einer

Halogenverbindung, die von einer bestimmten Probenmenge durch Anlagerung an C-C-Doppel- oder Dreifachbindungen aufgenommen werden kann. Die Bestimmung der Iodzahl ist dem Fachmann bekannt und wird durch genormte Analysemethoden, wie L 13.00-10 (entspricht DIN EN ISO 3961: 1999-08) oder Methode AOAC 26.020-26.021, bestimmt. Eine hohe Iodzahl steht daher für einen hohen Anteil an ungesättigten Bestandteilen in einer Verbindung.

Der Anteil an ungesättigten Fettsäuren in den Triglyceriden hat einerseits einen Einfluss auf die Pflege- und Färbeleistung der Mittel, andererseits jedoch auf die Stabilität der Zubereitungen, so dass für Triglyceridgemische (B) ein bestimmter, enger Iodzahl-Bereich eine optimale Balance zwischen den gewünschten und unerwünschten Eigenschaften darstellt.

Erfindungsgemäße Mittel enthalten daher pflanzliche Öle auf Basis von Fettsäuretriglyceridgemischen (B) mit einer Iodzahl von 50 bis 80, bevorzugt mit einer Iodzahl von 55 bis 75 und insbesondere bevorzugt mit einer Iodzahl von 60 bis 75.

Das Öl aus dem Fruchtfleisch von *Hippophae Rhamnoides* (Sanddorn) stellt ein erfindungsgemäß besonders geeignetes Fettsäuretriglyceridgemisch (B) dar. Das Öl zeichnet sich durch einen erhöhten Anteil an ungesättigten Fettsäuren aus und insbesondere von mehr als 30 Gew.-%, bezogen auf das Gesamtgewicht aller Fettsäuren im Triglyceridgemisch (B), an Palmitoleinsäure, so dass Sanddornfruchtfleischöl ein besonders bevorzugtes Fettsäuretriglyceridgemisch (B) darstellt. Sanddornfruchtfleischöl weist typischerweise einen Fettsäureschnitt, bestimmt mittels der voranstehend genannten Methoden, auf, der

| | |
|---|---|
| Palmitinsäure (C16:0) | zu mindestens ca. 30 Gew.-%, |
| Palmitoleinsäure (C16:1) | zu mindestens ca. 30 Gew.-%, |
| Oleinsäure (C18:1) | zu maximal ca. 30 Gew.-%, |
| Linolsäure (C18:2) | zu maximal ca. 20 Gew.-% und |
| Linolensäure (C18:3) | zu maximal ca. 5 Gew.-%, |

umfasst.

Eine weitere Ausführungsform der vorliegenden Erfindung ist daher dadurch gekennzeichnet, dass das Mittel als pflanzliches Öl auf Basis von Fettsäuretriglyceridgemisch (B) Sanddornfruchtfleischöl enthält.

Erfindungsgemäß bevorzugte Mittel sind dabei dadurch gekennzeichnet, dass das Mittel das/die pflanzliche(n) Öl(e) auf Basis eines Fettsäuretriglyceridgemischs (B) in einen Gewichtsanteil von 0,05 bis 10,0 Gew.-%, bevorzugt von 0,03 bis 5,0 Gew.-%, weiter bevorzugt 0,1 bis 3,5 Gew.-% und insbesondere von 0,50 bis 2,5 Gew.-%, jeweils bezogen auf das Gesamtgewicht des Mittels, enthält.

Neben dem triglyceridischen Anteil enthält Sanddornfruchtfleischöl weitere, unverseifbare Komponenten, die zur Erzielung der gewünschten Effekte beitragen und daher als weitere Zusätze besonders wünschenswert sind. Zu diesen Komponenten zählen insbesondere Carotinoide und fettlösliche Vitamine, insbesondere Vitamin A und Vitamin E.

Die erfindungsgemäßen Mittel können zur Nuancierung oder Farbintensivierung weitere farbgebenden Verbindungen enthalten, insbesondere ausgewählt aus Oxidationsfarbstoffvorprodukten und/oder direktziehenden Farbstoffen.

In einer weiteren Ausführungsform enthalten die erfindungsgemäßen Mittel alszusätzliche farbgebende Komponente mindestens ein Oxidationsfarbstoffvorprodukt.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate, einzusetzen.

Bevorzugte Entwicklerkomponenten werden ausgewählt aus aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäß bevorzugte Kupplerkomponenten werden ausgewählt aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die erfindungsgemäßen Mittel als farbgebende Komponente, insbesondere zur Nuancierung, mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 5 Gew.-%, bezogen auf das gesamte Mittel, eingesetzt. Die Gesamtmenge an direktziehenden Farbstoffen beträgt vorzugsweise höchstens 3 Gew.-%. Direktziehende Farbstoffe sind als anionische, kationische und nichtionische direktziehende Farbstoffe bekannt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Blue 347, Basic Brown 16 und Basic Brown 17, kationische Anthrachinonfarbstoffe wie HC Blue 16 (Bluequat B), sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Bevorzugt werden die erfindungsgemäßen Mittel als fließfähige Zubereitungen formuliert. Dabei sollten die Mittel so formuliert werden, dass das Mittel sich einerseits gut am Anwendungsort auftragen und verteilen lässt, andererseits jedoch ausreichend viskos ist, dass das Mittel während der Einwirkzeit am Wirkort verbleibt und nicht verläuft.

Es hat sich daher erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Bevorzugt handelt es sich bei dem Verdikkungsmittel um ein anionisches, synthetisches Polymer. Bevorzugte anionische Polymere sind Acrylsäure- und/oder

Methacrylsäure-Polymere oder -Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine $C_1$-$C_4$-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogenen Monomereinheiten, bevorzugt Acrylamid, Methacrylamid, Acrylsäure-$C_1$-$C_4$-Alkylester und Methacrylsäure-$C_1$-$C_4$-Alkylester, eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der Erfindung erwiesen. Insbesondere Tone, vorzugsweise Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Besonders vorteilhafte Verdickungsmittel sind dabei solche, die neben der Verdickungsleistung zusätzlich einen Beitrag zu den gewünschten Eigenschaften der Mittel, wie beispielsweise der Pflegevermögen, leisten. Es zeigte sich, dass gerade amphotere Polymere diese Eigenschaft in besonderem Maße aufweisen. In einer bevorzugten Ausführungsform enthalten die erfindungsgemäßen Mittel jedoch zusätzlich mindestens ein amphoteres Polymer. Dabei kann das amphotere Polymer durch Polymerisation ethylenischer Monomere aufgebaut sein, wobei sowohl aus verschiedenen, kationische Gruppen tragenden Monomeren sowie verschiedenen, anionische Gruppen tragenden Monomeren und gegebenenfalls auch nichtionische Monomeren eingesetzt werden können. Bevorzugte kationische Monomere sind dabei Dialkyldiallylammonium-Verbindungen, quaternäre Alkyl-Vinyl-Imidazolium-Verbindungen sowie (Meth-)Acrylsäureester oder (Meth-)-Acrylsäureamide mit Trialkylammonioalkyl-Resten. Bevorzugte anionische Monomere sind dabei physiologisch verträgliche Salze der Acrylsäure oder Methacrylsäure. Geeignete, gegebenenfalls zusätzlich einzusetzende nichtionische Monomere sind Vinylcaprolactam, Vinylpyrrolidinon, Vinylimidazol, Acrylamid und Methacrylamid.

Besonders bevorzugte amphotere Polymere werden unter der INCI-Bezeichnung "Acrylamidopropyltrimonium Chloride/Acrylates Copolymer" vertrieben.

Die amphoteren Polymere werden in bevorzugt in einer Gesamtmenge von 0,01 bis 5 Gew.%, bevorzugt 0,05 bis 3 Gew.% und ganz besonders bevorzugt von 0,1 bis 2 Gew.%, bezogen auf die Gesamtmenge des anwendungsbereiten Mittels, eingesetzt.

Üblicherweise enthalten die erfindungsgemäßen Mittel zusätzlich als oberflächenaktive Substanz ein Emulgator bzw. ein Tensid, wobei oberflächenaktive Substanzen je nach Anwendungsgebiet als Tenside oder als Emulgatoren bezeichnet werden. Bevorzugt sind dabei insbesondere anionischen und nichtionischen Tenside und Emulgatoren.

Eine weitere Ausführungsform der vorliegenden Erfindung ist daher dadurch gekennzeichnet, dass das Mittel zusätzlich mindestens ein nichtionisches und/oder anionisches Tensid enthält.

Als anionische Tenside eignen sich in erfindungsgemäßen Zubereitungen alle für die Verwendung am menschlichen Körper geeigneten anionischen oberflächenaktiven Stoffe.

Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 6 bis 20 C-Atomen in der Alkylgruppe und bis zu 12 Glykolethergruppen im Molekül.

Als zusätzliches anionisches Tensid enthalten die Mittel bevorzugt mindestens eine Ethercarbonsäure der Formel $RO(CH_2CH_2O)_xCH_2COOH$ und/oder eines ihrer physiologisch verträglichen Salze, worin R für eine $C_6$-$C_{20}$-Alkylkette steht und x = 0 oder eine Zahl 1 bis 16 ist.

Beispiele bevorzugter Verbindungen vom Typ der polyalkoxylierten Ethercarbonsäuren werden unter den Handelsnamen Akypo TEC (INCI-Bezeichnung: Oleth-10 Carboxylic Acid, Capryleth-9 Carboxylic Acid, Hexeth-4 Carboxylic Acid), Akypo RLM 38 (INCI-Bezeichnung: LAURETH-5 Carboxylic Acid), Akypo RO 90 (INCI-Bezeichnung: Oleth-10 Carboxylic Acid), Akypo Soft 100 (INCI-Bezeichnung: Sodium Laureth-11 Carboxylate), Akypo RO 20 (INCI-Bezeichnung: Oleth-3 Carboxylic Acid), Akypo Soft 45 HP (INCI-Bezeichnung: Sodium Laureth-6 Carboxylate) oder Akypo RLM 45 (INCI-Bezeichnung: Laureth-6 Carboxylic Acid) vertrieben.

Die anionischen Tenside werden in bevorzugt in einer Gesamtmenge von 0,1 bis 30 Gew.%, bevorzugt 1 bis 25 Gew.% und ganz besonders bevorzugt von 1 bis 15 Gew.%, bezogen auf die Gesamtmenge des anwendungsbereiten Mittels, eingesetzt.

Als besonders vorteilhaft haben sich außerdem oberflächenaktive Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30, sowie Alkylpolyglucoside entsprechend der allgemeinen Formel RO-$(Z)_n$, wobei R für Alkyl, Z für Zucker sowie n für die Anzahl der Zuckereinheiten steht.

Als zusätzliches nichtionisches Tensid enthalten die Mittel bevorzugt mindestens ein Alkylpolyglucosid der Formel R'O-$(Z)_n$, worin R' für eine $C_{10}$-$C_{20}$-Alkylkette, Z für einen Zuckerrest mit 5 oder 6 Kohlenstoffatomen und n für eine Zahl von 1 bis 15 steht. Beispiele solcher Verbindungen tragen die INCI-Bezeichnungen Decyl Glucoside, Lauryl Glucoside, Cetearyl Glucoside und Coco-Glucoside.

Erfindungsgemäß bevorzugte Mittel enthalten als nichtionisches Tensid das Anlagerungsprodukt von Cocos-Fettalkohol an (Poly-)glucose, welches unter der INCI-Bezeichnung Coco-Glucoside bekannt ist. Ein solches Produkt wird unter anderem unter dem Handelsnamen Lamesoft PO 65 (INCI-Bezeichnung: Coco-Glucoside, Glyceryl Oleate, Aqua) vertrieben.

Die nichtionischen Tenside werden in bevorzugt in einer Gesamtmenge von 0,1 bis 30 Gew.%, bevorzugt 1 bis 20 Gew.% und ganz besonders bevorzugt von 1 bis 10 Gew.%, bezogen auf die Gesamtmenge des anwendungsbereiten Mittels, eingesetzt.

Eine weitere Ausführungsform der vorliegenden Erfindung ist daher dadurch gekennzeichnet, dass dass das Mittel zusätzlich mindestens ein nichtionisches Tensid,
ausgewählt aus Alkylpolyglucosiden der Formel R'O-$(Z)_n$, worin R' für eine $C_{10}$-$C_{20}$-Alkylkette, Z für einen Zuckerrest mit 5 oder 6 Kohlenstoffatomen und n für eine Zahl von 1 bis 15 steht, und/oder zusätzlich mindestens ein anionisches Tensid,
ausgewählt aus Ethercarbonsäuren der Formel $RO(CH_2CH_2O)_xCH_2COOH$ und/oder einem ihrer physiologisch verträglichen Salze, worin R für eine $C_6$-$C_{20}$-Alkylkette steht und x = 0 oder eine Zahl 1 bis 16 ist,
enthält.

Die erfindungsgemäßen Mittel können weitere Hilfs- und Zusatzstoffe enthalten. Bevorzugt enthalten die fließfähigen Zubereitungen zusätzlich als weitere oberflächenaktive Substanz ein kationisches, amphoteres und/oder zwitterionisches Tensid.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Die zwitterionischen und amphoteren Tenside werden in bevorzugt in einer Gesamtmenge von 0,1 bis 15 Gew.%, bevorzugt 0,5 bis 10 Gew.% und ganz besonders bevorzugt von 1 bis 5 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise nichtionische Polymere, kationische Polymere, zwitterionische Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, $B_3$, $B_5$, $B_6$, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Komplexbilnder, wie EDTA oder Etidronsäure, Perlglanzmittel wie Ethylenglykolmono- und -distearat sowie PEG-3-distearat, Pigmente, Aufhellverstärker (wie Natriumpersulfat, Kaliumpersulfat oder Ammoniumpersulfat), Treibmittel wie Propan-Butan-Gemische, $N_2O$, Dimethylether, $CO_2$ und Luft und Antioxidantien.

Die Auswahl dieser weiteren Stoffe wird der Fachmann gemäß der gewünschten Eigenschaften der Mittel

treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Zur verbesserten Farbausbildung und möglichst umfassender Faserschonung besitzen die Mittel einen alkalischen pH-Wert von 8 bis 10,5. Eine weitere bevorzugte Ausführungsform der vorliegenden Erfindung besteht darin, dass das anwendungsbereite Mittel einen pH-Wert zwischen 8,5 und 10,0, bevorzugt zwischen 9,0 und 9,8, insbesondere bevorzugt zwischen 9,2 und 9,5 besitzt. Bei den pH-Werten im Sinne der vorliegenden Erfindung handelt es sich um pH-Werte, die bei einer Temperatur von 22°C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol, Triethanolamin, Ammoniak, 1-Aminopropan-2-ol und 2-Amino-2-methylpropan-1-ol.

Es hat sich gezeigt, dass der Zusatz von basischen Aminosäuren nicht nur zur pH-Wert-Einstellung günstig ist, sondern dass gleichermassen eine zusätzliche Pflege und Verbesserung der Faserstruktur, insbesondere an Faseroberfläche einhergeht. Basische Aminosäuren im Sinne der Erfindung sind insbesodnere Arginin, Histidin, Lysin und Ornithin.

Eine weitere Ausführungsform des ersten Erfindungsgegenstands ist daher ein Mittel, welches dadurch gekennzeichnet ist, dass das Mittel zusätzlich mindestens eine basische Aminosäure, bevorzugt Arginin, und/oder eines deren physiologisch verträglichen Salze enthält.

Die Konfektionierung der erfindungsgemäßen Farbveränderungsmittel unterliegt prinzipiell keinerlei Beschränkungen. Die erfindungsgemäßen Mittel können dabei bevorzugt als 1-Komponentenmittel konfektioniert werden.

Der erfindungsgemäßen Mittel werden in Verfahren zur Färbung keratinischer Fasern, insbesondere menschlicher Haare und bevorzugt menschlicher Kopfhaare am lebenden Anwender, eingesetzt.

Solche Verfahren zur Luftsauerstoff induzierten Farbveränderung menschlicher Haare sind dadurch gekennzeichnet, dass ein erfindungsgemäßes Mittel gemäß obiger Vorgaben auf das Haar aufgetragen wird, für eine Einwirkzeit auf dem Haar belassen wird, und anschließend das Haar ausgespült wird.

Ein weiterer Gegenstand der vorliegenden Erfindung ist daher ein Verfahren zur durch Luftsauerstoff induzierten Färbung von keratinhaltigen Fasern, insbesondere menschlichen Haaren, bei dem ein Mittel des ersten Erfindungsgegenstand auf die Fasern aufgebracht werden, für einen Zeitraum von 2 bis 120 min, bevorzugt von 3 bis 45 min, weiter bevorzugt von 10 bis 40 min, auf den Fasern belassen wird und anschließend ausgespült wird.

Die Anwendungstemperaturen bei der erfindungsgemäßen Farbveränderung keratinischer Fasern können in einem Bereich zwischen 15 und 45°C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Bezüglich weiterer bevorzugter Ausführungsformen der erfindungsgemäßen Verfahren gilt mutatis mutandis das zu den erfindungsgemäßen Mitteln Gesagte.

Durch Anwendung der erfindungsgemäßen Mittel zur Luftsauerstoff induzierten Färbung menschlicher Haare wird eine verbesserte Haaroberfläche gegenüber vergleichbaren Färbemitteln erzielt. Ebenso werden eine vergleichsweise verringerte Haarstrukturschädigung und damit eine verbesserte Pflege der Haarfasern beobachtet. Daneben zeichnen sich mit den erfindungsgemäßen

Mitteln behandelten Fasern neben der Färbung auch durch einen verbesserten Glanz aus.

Ein weiterer Erfindungsgegenstand ist daher die Verwendung der Mittel des ersten Erfindungsgegenstandes zur Verbesserung des Glanzes keratinhaltiger Fasern, insbesondere menschlicher Haare, bei durch Luftsauerstoff induzierten Färbungen keratinhaltiger Fasern, insbesondere menschlicher Haare.

Bezüglich weiterer bevorzugter Ausführungsformen der erfindungsgemäßen Verwendungen gilt mutatis mutandis das zu den erfindungsgemäßen Mitteln Gesagte.

<u>Beispiele</u> (alle Mengenangaben in Gew.-%)

**1) Färbemittel**

(Tabelle 1)

| Rohstoff | V1 | V2 | E1 | E2 |
|---|---|---|---|---|
| Cetearyl Alcohol | 10,00 | 10,00 | 10,00 | 8,00 |
| $C_{12}$-$C_{18}$-Fettalkohol | 2,40 | 2,40 | 2,40 | 3,00 |
| Ceteareth-12 | 0,50 | 0,50 | 0,50 | 0,50 |
| Ceteareth-20 | 0,50 | 0,50 | 0,50 | 0,50 |
| Akypo Soft 45HP [1] | 10,00 | 10,00 | 10,00 | 10,00 |
| Texapon K 14 S Special, 70 % [2] | 2,80 | 2,80 | 2,80 | 2,80 |
| Plantacare 1200 UP [3] | 2,00 | 2,00 | 2,00 | 2,00 |
| 2-Octyldodecanol | 1,00 | 1,00 | 1,00 | 1,00 |
| L-Arginin | 0,50 | 0,50 | 0,50 | 1,10 |
| 5,6-Dihydroxyindolin HBr | 0,95 | 0,95 | 0,95 | 1,60 |
| 2-Methylresorcin | --- | --- | --- | 0,50 |
| Ascorbinsäure | 0,20 | 0,20 | 0,20 | 0,20 |
| Sanddornfruchtfleischöl | --- | --- | 1,00 | 1,00 |
| Marulaöl [4] | --- | 1,00 | --- | --- |
| Ammoniak, 25 % | 0,40 | 0,40 | 0,40 | 0,40 |
| Parfüm | qs | qs | qs | qs |
| Wasser, entsalzt | | Ad | 100 | |

[1] $C_{12}$-$C_{14}$-Alkylether, ethoxyliert (6 EO) Carbonsäure, Natriumsalz (ca. 21 %, INCI-Bezeichnung: Sodium Laureth-6 Carboxylate, Aqua) (KAO)
[2] $C_{12}$-$C_{14}$-Alkylethersulfat, ethoxyliert (3 EO), Natriumsalz (ca. 70 %, INCI-Bezeichnung: Sodium Myreth Sulfate, Aqua) (BASF)
[3] $C_{12}$-$C_{16}$-Alkylpolyglucosid (ca. 51 %, INCI-Bezeichnung: Lauryl Glucoside, Aqua) (BASF)
[4] Marulaöl: pflanzliches Öl auf Basis eines Fettsäuretriglyceridgemsiches mit Palmitoleinsäuregehalt von ca. 0,1 Gew.-%, bezogen auf das Gesamtgewicht aller Fettsäuren des Fettsäuretriglycerids

Rezepturen V1 ohne pflanzliches Öl und V2 mit einem pflanzlichen Öl, welches einen Palmitoleinsäuregehalt unter 25 Gew.-% aufweist, sind nicht erfindungsgemäße Vergleichsrezepturen. E1 und E2 sind erfindungsgemäße Mittel.

**2) Ausfärbungen**

Die unter 1) beschriebenen Färbemittel wurden jeweils auf 3 Strähnen (Kerling International, Backnang (DE); europäisches, natürliches Humanhaar 9/0) angewendet. Pro Gramm Haarsträhne wurden 4 g der jeweiligen Färbemittel aufgetragen. Die Einwirkzeit betrug 30 min bei 35°C für die Färbemittel. Danach wurden die Strähnen 90 s lang mit warmem Wasser ausgespült und luftgetrocknet.

**3) Glanzmessungen**

Die Haarsträhnen wurden jeweils vor und nach der Behandlung in einer Glanzapparatur auf beiden Seiten vermessen, so dass pro Strähne 2 Meßpunkte erhalten wurden.

Bei der Glanzapparatur handelt es sich um eine mit einer lichtabsorbierenden Auskleidung versehene Glanzkammer. Die zu vermessenden Haarsträhnen wurden auf einen Zylinder gespannt und in der Mitte der Glanzkammer positioniert. Oberhalb des Zylinders befindet sich als Beleuchtungsquelle eine stabförmige Gasentladungslampe, welche die auf dem Zylinder aufgespannte Haarsträhne durch eine kleine Blende beleuchtet. Mit einer Digitalkamera wurde die Glanzkurve jeder Strähne vermessen und bildanalytisch ausgewertet. Anschließend wurde der Glanzwert der Strähnen (L) nach der Formel nach Reich und Robbins (1) berechnet:

$$L_{Reich/Robbins} = \frac{S}{D * W\frac{1}{2}}$$

L: Glanz
S: gerichtete Reflexion
D: diffuse Reflexion
W½: Halbwertsbreite der Glanzkurve
(1) C. Reich and C.C. Robbins, J. Soc. Cosmet. Chem. 44, 221-234 (1993)

Die Veränderung des Glanzes wurde durch Vergleich der vor und nach der Applikation des erfindungsgemäßen Mittels gemessenen Glanzwerte bewertet. Mittels statistischer Verfahren wurde berechnet, ob die Änderung des Glanzes signifikant ist.

**4) Ergebnisse der Glanzmessung** (Tabelle 2)

Folgende Messergebnisse wurden erhalten.

| Färbezubereitung | Unterschied in % gegenüber V1 |
|---|---|
| V1 | --- |
| V2 | +/- 0 % |
| E1 | + 19,5% |

Aus den Ergebnissen aus Tabelle 2 wird deutlich, dass der Zusatz eines pflanzlichen Öls auf Basis von Fettsäuretriglyceridgemisch (B) (erfindungsgemäßes Mittel E1) eine signifikante Glanzverbesserung gegenüber dem Vergleichsmittel V1. Es zeigt sich weiterhin, dass der Zusatz von pflanzlichen Ölen auf Basis von Fettsäuretriglyceridgemischen ohne den erfindungswesentlichen Palmitoleingehalt (Mittel V2) zu keiner Veränderung des Glanzes führt.

## Patentansprüche

1. Mittel zur durch Luftsauerstoff induzierten Färbung keratinhaltiger Fasern, insbesondere menschlicher Haare, welches in einem kosmetisch akzeptablen Träger bei einem alkalischen pH-Wert von 8 bis 10,5

(a) mindestens eine Verbindung der Formel (I),

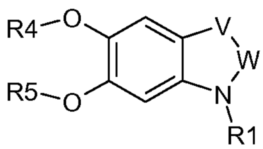

(I),

worin unabhängig voneinander

- R1 für Wasserstoff, eine $C_1$-$C_4$-Alkylgruppe oder eine $C_1$-$C_4$-Hydroxyalkylgruppe steht,
- V für CHR3 und W für CHR2 stehen,
so dass die Bindung zwischen V und W einer Einfachbindung entspricht,
oder V für CR3 und W für CR2 stehen,
so dass die Bindung zwischen V und W einer Doppelbindung entspricht,
wobei jeweils
- R2 für Wasserstoff oder eine -COOH-Gruppe steht, wobei die -COOH-Gruppe auch als Salz mit einem physiologisch verträglichen Kation vorliegen kann,
- R3 für Wasserstoff oder eine $C_1$-$C_4$-Alkylgruppe steht,

- R4 und R5 unabhängig voneinander für Wasserstoff, eine $C_1$-$C_4$-Alkylgruppe oder eine Gruppe -CO-R6 stehen, in der R6 für eine $C_1$-$C_4$-Alkylgruppe steht,

und/oder eines deren physiologisch verträgliche Salze mit einer organischen oder anorganischen Säure,

(b) und mindestens ein pflanzliches Öl auf Basis eines Fettsäuretriglyceridgemischs (B) enthält,
**dadurch gekennzeichnet, dass**
neben Luftsauerstoff kein zusätzliches Oxidationsmittel enthalten ist, und dass das Fettsäuretriglyceridgemisch (B) einen Anteil von mindestens 25 Gew.-% Palmitoleinsäure, bezogen auf Gesamtgewicht aller Fettsäuren des Fettsäuretriglyceridgemisches (B), aufweist.

**2.** Mittel nach Anspruch 1, **dadurch gekennzeichnet, dass** die Verbindungen der Formel (I) ausgewählt werden aus Verbindungen der Formel (Ia),

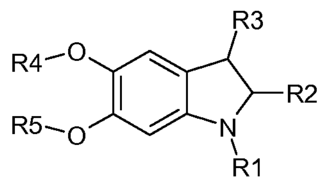

(Ia),

worin unabhängig voneinander

- R1 für Wasserstoff, eine $C_1$-$C_4$-Alkylgruppe oder eine $C_1$-$C_4$-Hydroxyalkylgruppe steht,
- R2 für Wasserstoff oder eine -COOH-Gruppe steht, wobei die -COOH-Gruppe auch als Salz mit einem physiologisch verträglichen Kation vorliegen kann,
- R3 für Wasserstoff oder eine $C_1$-$C_4$-Alkylgruppe steht,
- R4 und R5 unabhängig voneinander für Wasserstoff, eine $C_1$-$C_4$-Alkylgruppe oder eine Gruppe -CO-R6 stehen, in der R6 für eine $C_1$-$C_4$-Alkylgruppe steht,

und/oder deren physiologisch verträglichen Salzen mit einer organischen oder anorganischen Säure.

**3.** Mittel nach einem der Ansprüche 1 oder 2, **dadurch gekennzeichnet, dass** die Verbindungen der Formel (I) ausgewählt werden aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure und/oder deren physiologisch verträglichen Salzen mit einer organischen oder anorganischen Säure.

**4.** Mittel nach einem der Ansprüche 1 bis 3, **dadurch gekennzeichnet, dass** die Verbindungen der Formel (I) in einem Anteil von 0,01 bis 10 Gew.-%, bevorzugt von 0,1 bis 2,0 Gew.-% und besonders bevorzugt von 0,6 bis 1,2 Gew.-%, jeweils bezogen auf das Gesamtgewicht des Mittels, enthalten sind.

**5.** Mittel nach einem der Ansprüche 1 bis 4, **dadurch gekennzeichnet, dass** das Mittel als pflanzliches Öl auf Basis von Fettsäuretriglyceridgemisch (B) Sanddornfruchtfleischöl enthält.

**6.** Mittel nach einem der Ansprüche 1 bis 5, **dadurch gekennzeichnet, dass** das Mittel das/die pflanzliche(n) Öl(e) auf Basis eines Fettsäuretriglyceridgemischs (B) in einen Gewichtsanteil von 0,05 bis 10,0 Gew.-%, bevorzugt von 0,03 bis 5,0 Gew.-%, weiter bevorzugt 0,1 bis 3,5 Gew.-% und insbesondere von 0,50 bis 2,5 Gew.-%, jeweils bezogen auf das Gesamtgewicht des Mittels, enthält.

**7.** Mittel nach einem der Ansprüche 1 bis 6, **dadurch gekennzeichnet, dass** das Mittel zusätzlich mindestens ein nichtionisches Tensid,
ausgewählt aus Alkylpolyglucosiden der Formel $R'O-(Z)_n$, worin R' für eine $C_{10}$-$C_{20}$-Alkylkette, Z für einen Zuckerrest mit 5 oder 6 Kohlenstoffatomen und n für eine Zahl von 1 bis 15 steht, und/oder zusätzlich mindestens ein anionisches Tensid,
ausgewählt aus Ethercarbonsäuren der Formel $RO(CH_2CH_2O)_xCH_2COOH$ und/oder einem ihrer physiologisch verträglichen Salze, worin R für eine $C_6$-$C_{20}$-Alkylkette steht und x = 0 oder eine Zahl 1 bis 16 ist,
enthält.

**8.** Mittel nach einem der Ansprüche 1 bis 7, **dadurch gekennzeichnet, dass** das Mittel zusätzlich mindestens eine basische Aminosäure, bevorzugt Arginin, und/oder eines deren physiologisch verträglichen Salze enthält.

**9.** Verfahren zur durch Luftsauerstoff induzierten Färbung von keratinhaltigen Fasern, insbesondere menschlichen Haaren, bei dem ein Mittel nach einem Ansprüche 1 bis 8 auf die Fasern aufgebracht werden, für einen Zeitraum von 2 bis 120 min, bevorzugt von 3 bis 45 min, weiter bevorzugt von 10 bis 40 min, auf den Fasern belassen wird und anschließend ausgespült wird.

**10.** Verwendung eines Mittels nach einem der Ansprüche 1 bis 8 zur Verbesserung des Glanzes keratinhaltiger Fasern, insbesondere menschlicher Haare, bei durch Luftsauerstoff induzierten Färbungen keratinhaltiger Fasern, insbesondere menschlicher Haare.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **C. REICH ; C.C. ROBBINS.** *J. Soc. Cosmet. Chem.,* 1993, vol. 44, 221-234